# EUROPEAN PATENT APPLICATION

(11) **EP 4 628 591 A1**
(43) Date of publication of application: **08.10.2025**
(21) Application number: 23898208.6
(22) Date of filing: 24.11.2023
(51) Int. Cl.: C12N 15/82, C12N 9/22, C12N 15/113

(54) **METHOD FOR PRODUCING CAAIBZ1 GENE-EDITED PEPPER TO IMPROVE DROUGHT TOLERANCE**

(30) Priority: 01.12.2022 KR 20220166094
(71) Applicant: Toolgen Incorporated, Seoul 07789 (KR)
(72) Inventor: WOO, Je Wook, Seoul 07789 (KR); HARN, Chee Hark, Seoul 07789 (KR); KANG, Min Sung, Seoul 07789 (KR); MOON, Ah Ram, Seoul 07789 (KR); SUNG, Eun Ji, Seoul 07789 (KR); PARK, Yunji, Seoul 07789 (KR)
(74) Representative: Ipsilon
(86) International application number: PCT/KR2023/019049
(87) International publication number: WO 2024/117677

(57) **Abstract**

The present invention relates to a genome editing composition for enhancing drought tolerance in hot pepper plant and a method of producing T-DNA free genome-edited hot pepper plant with enhanced drought tolerance by using the composition. More specifically, the composition of the present invention comprises, as an active ingredient, a complex (i.e., ribonucleoprotein) of a guide RNA specific to a target nucleotide sequence of the *CaAIBZ1* gene derived from hot pepper and an endonuclease protein; or a recombinant vector containing a DNA encoding a guide RNA specific to a target nucleotide sequence of the *CaAlBZ1* gene and a nucleic acid sequence encoding an endonuclease protein.

## Description

### TECHNICAL FIELD

The present invention relates to a method for producing *CaAIBZ1* (*Capsicum annuum* ASRF 1-Interacting bZIP transcription factor 1) gene-edited hot pepper to enhance drought tolerance.

This work was supported by the New Breeding Technology Center of the Rural Development Administration of South Korea (Proj ect Number: PJ01479802).

### BACKGROUND ART

Due to climate change and global warming, natural disasters such as droughts, water shortages, and floods are occurring worldwide, and these phenomena will become more severe in the future. Hot peppers (*Capsicum annuum L*.) make up the second largest vegetable market in the world after tomatoes and are cultivated worldwide. More than 90% of sweet peppers or chili peppers are grown in open fields, and in countries such as India and China where hot pepper cultivation is particularly dense, production can drop sharply during the growing season due to droughts and water shortages. Currently, there are no genetic resources for hot peppers that are drought-tolerant, so it is difficult to develop varieties that can adapt to various environmental changes such as drought using existing traditional breeding and MAS (maker-assisted selection) breeding. On the other hand, gene editing technology can lead to the development of new genetic resources, making it possible to develop drought-tolerant hot peppers.

In the present invention, a guide RNA for a target gene that confers drought tolerance is inserted into a Cas9 expression vector along with GFP (green fluorescent protein), and a gene-edited strain is developed through hot pepper transformation, and then a drought-tolerant hot pepper is selected.

Meanwhile, Korean Patent Publication No. 2015-0106528 discloses *'MSRB2* gene derived from hot pepper for increasing abiotic stress tolerance of plants and use thereof, and Korean Patent Publication No. 2013-0055050 discloses '*CaWRKY1* gene, a transcription factor responsible for resistance to both cold damage and drought in hot pepper, and use thereof, but the 'Method for producing *CaAIBZ1* gene-edited hot pepper to enhance drought tolerance' of the present invention is not described.

### DETAILED DESCRIPTION OF INVENTION

### TECHNICAL PROBLEMS TO BE SOLVED

The present invention is devised in view of the need described above, and the inventors of the present invention constructed a CRISPR vector including a gRNA targeting *CaAIBZ1,* a gene related to drought tolerance, and Cas9 and GFP expression cassettes. Thereafter, the vector was transformed into hot pepper cotyledon explants via *Agrobacterium,* and genome-edited plants in which the target region is edited were obtained. Thereafter, a drought tolerance test employing both water withholding and re-irrigation was performed using the T₂ generation genome-edited plants, and it was consequently found that bi-allelic *CaAIBZ1* gene-edited plants exhibited significantly increased drought tolerance compared to the control group (non-edited plant) and mono-allelic edited plants, thereby completing the present invention.

### TECHNICAL MEANS FOR SOLVING THE PROBLEMS

To solve the problems that are described in the above, the present invention provides a genome editing composition for enhancing drought tolerance in hot pepper plant, comprising as an active ingredient: a complex (i.e., ribonucleoprotein) of a guide RNA specific to a target nucleotide sequence of the *CaAIBZ1* (*Capsicum annuum* ASRF1-Interacting bZIP transcription factor 1) gene derived from hot pepper and an endonuclease protein; or a recombinant vector containing a DNA encoding a guide RNA specific to a target nucleotide sequence of the *CaAIBZ1* gene and a nucleic acid sequence encoding an endonuclease protein.

The present invention further provides a method for producing a genome-edited hot pepper plant with enhanced drought tolerance, comprising: (a) introducing a guide RNA specific to a target nucleotide sequence of the *CaAIBZ1* gene derived from hot pepper and an endonuclease protein into a hot pepper plant cell to edit the genome; and (b) regenerating a hot pepper plant from the genome-edited hot pepper plant cell.

The present invention still further provides a genome-edited hot pepper plant with enhanced drought tolerance that is produced by the above method, and a genome-edited seed thereof.

### ADVANTAGEOUS EFFECT OF INVENTION

The genome-edited hot pepper plant with enhanced drought tolerance produced by the method of the present invention is expected to be useful for securing genetic resources of drought-tolerant hot pepper and developing high-value hot pepper varieties in the future. Additionally, since the method of the present invention does not involve the insertion of foreign genes and only involves small mutations that cannot be distinguished from natural variations, it is anticipated that it will save both cost and time compared to GMO (Genetically Modified Organism) crops, which require significant costs and time for evaluation of safety and environmental impact.

### BRIEF DESCRIPTION OF THE DRAWINGS

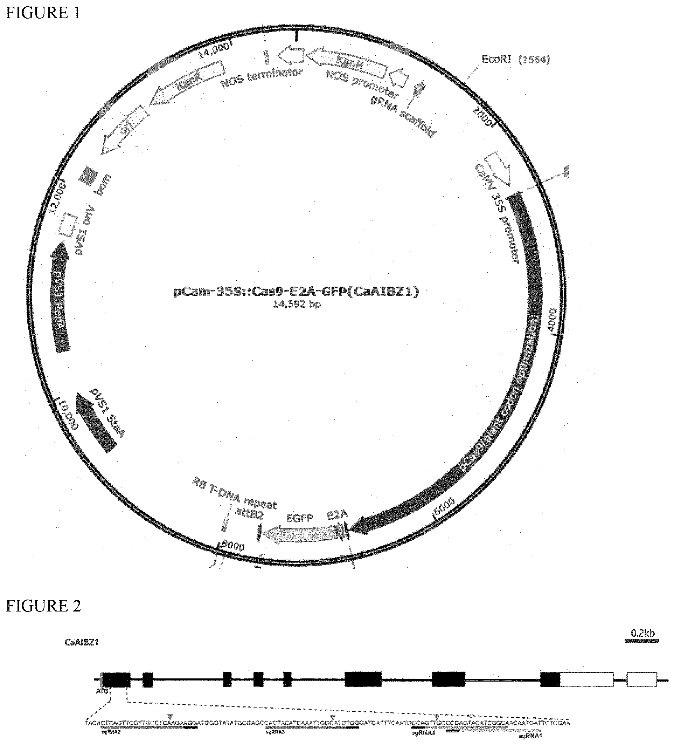

Figure 1 shows a schematic diagram of the CRISPR-Cas9 vector pCam-CaAIBZ1 for gene editing of the hot pepper *CaAIBZ1* gene. The hot pepper gene-editing vector has a pCambia vector backbone and a structure of simultaneously containing the gRNA expression and Cas9 expression cassette and GFP expression cassette. Cas9 expression is regulated by the Cauliflower mosaic virus (CaMV) 35S promoter, while the gRNA targeting the *CaAIBZ1* gene is regulated by the AtU6 promoter. LB: T-DNA left border; NLS: nuclear localization signal; pCas9: Plant-codon optimized spCas9; 2A: 2A self-cleaving peptide; eGFP: enhanced green fluorescent protein; CaMV35S P: Cauliflower mosaic virus (CaMV) 35S promoter; 35S(T): CaMV 35S terminator, NOS-T: nopaline synthase terminator; NPTII: neomycin phosphotransferase II (kanamycin resistance determinants), RB: T-DNA right border.
Figure 2 shows the position and sequence of the target guide RNA of the *CaAIBZ1* gene on the hot pepper genome. Specifically, the color bar represents the target RNA position, the black bar indicates the PAM (Protospacer Adjacent Motif) site, the arrow marks the predicted cleavage site, and the black box highlights the exon region on the genome.
Figure 3 shows the analysis results of the editing efficiency of the target gRNA using RNP (sgRNA/Cas9 protein) in hot pepper protoplasts.
Figure 4 illustrates the process of obtaining hot pepper transgenic plants from cotyledon explants through the callus stage, followed by regeneration and growth into small plantlets.
Figure 5 shows the appearance of *CaAlBZ1* gene-edited hot pepper plants (T₀) in the flowering stage in a plant growth chamber, after pot planting.
Figure 6 shows the NGS results analyzing the editing pattern of the *CaAIBZ1* gene-edited T₀ hot pepper plants.
Figures 7 and 8 show the NGS results analyzing the editing pattern of the *CaAIBZ1* gene-edited T₁ hot pepper plants.
Figure 9 shows the *CaAIBZ1* gene-edited T₁ generation hot pepper plants, which are used to obtain T₂ seeds for drought tolerance tests.
Figure 10 shows results of the drought tolerance test for the *CaAlBZ1* gene-edited T₂ hot pepper plants.
Figure 11 shows results of the survival rate test carried out after re-watering for the *CaAlBZ1* gene-edited T₂ hot pepper plants.
Figure 12 shows the results of PCR analysis for determining the T-DNA-free status of the *CaAIBZ1* gene-edited T₂ hot pepper plants. M: 1kb marker, N: wild type, P: vector, 10-1 #1~12-18 #1: *CaAIBZ1* gene edited hot pepper T₂ plants.

### BEST EMBODIMENT(S) FOR CARRYING OUT INVENTION

To achieve object of the present invention, the present invention provides a genome editing composition for enhancing drought tolerance in hot pepper plant, comprising as an active ingredient: a complex (i.e., ribonucleoprotein) of a guide RNA specific to a target nucleotide sequence of the *CaAIBZ1* (*Capsicum annuum* ASRF1-Interacting bZIP transcription factor 1) gene derived from hot pepper and an endonuclease protein; or a recombinant vector containing a DNA encoding a guide RNA specific to a target nucleotide sequence of the *CaAIBZ1* gene and a nucleic acid sequence encoding an endonuclease protein.

In the composition of the present invention, the target gene for genome editing to enhance drought tolerance in hot pepper plants is the *CaAIBZ1* gene, with GenBank accession number LY715037.1.

In this specification, the term "genome/gene editing" refers to a technique that enables the introduction of targeted mutations into the plant and animal cells, including human cells, and it is a technique allowing the knock-out or knock-in of specific genes according to the creation of deletions, insertions, or substitutions in the nucleic acid molecules through DNA cleavage, or the introduction of mutations even in non-coding DNA sequences that do not produce any protein. The term "gene editing" may be used interchangeably with "gene modification."

For the purposes of the present invention, the genome editing may specifically involve introducing mutations into the plant by using endonucleases, such as the Cas9 (CRISPR-associated protein 9) protein, and guide RNA.

Furthermore, the term "target gene" refers to a specific DNA sequence within the genome of the plant that is to be edited in the present invention, and it may include both coding and non-coding regions. Based on the specific objectives, a person who is skilled in the pertinent art can select the target gene for the genome-edited plants to be produced.

Furthermore, the term "guide RNA" refers to a short, single-stranded RNA that contains an RNA sequence specific to the target DNA in the nucleotide sequence encoding the target gene. The guide RNA means a ribonucleic acid which binds complementarily to all or part of the target DNA sequence, guiding the endonuclease protein to the target DNA sequence. The guide RNA can be in the form of dual RNA, comprising two RNAs: crRNA (CRISPR RNA) and tracrRNA (trans-activating CRISPR RNA) as a constitutional element, or single-strand guide RNA (sgRNA), comprising a first region containing a sequence complementary to all or part of the target gene's nucleotide sequence and a second region that interacts with the endonuclease (particularly RNA-guided nucleases). As long as the endonuclease can be active at the target nucleotide sequence, any form may be included within the scope of the present invention. The guide RNA may be prepared and used according to the known techniques in the field, considering the type of endonuclease used or the microorganism from which the endonuclease is derived.

Additionally, the guide RNA may be those transcribed from a plasmid template, those transcribed *in vitro* (e.g., as oligonucleotide double strand), or those synthesized as a guide RNA, but it is not limited to these forms.

In the genome editing composition according to the present invention, the guide RNA is specifically designed to target the nucleotide sequence of the hot pepper-derived *CaAIBZ1* gene, which consists of the nucleotide sequences of SEQ ID NO: 1, SEQ ID NO: 3, or SEQ ID NO: 4.

Furthermore, in the genome editing composition according to the present invention, the endonuclease protein may be one or more selected from the group consisting of Cas9, Cpf1 (CRISPR from *Prevotella* and *Francisella* 1, also known as CAs12a), TALEN (Transcription activator-like effector nuclease), ZFN (Zinc Finger Nuclease), or functional equivalents thereof. Preferably, the endonuclease protein is Cas9 protein, but it is not limited to this.

Additionally, the Cas9 protein may be one or more selected from the group consisting of Cas9 protein derived from *Streptococcus pyogenes,* Cas9 protein derived from *Campylobacter jejuni,* Cas9 protein derived from *Streptococcus thermophilus* or *Staphylococcus aureus,* Cas9 protein derived from *Neisseria meningitidis,* Cas9 protein derived from *Pasteurella multocida,* Cas9 protein derived from *Francisella novicida,* and others, but it is not limited to these. The Cas9 protein or its genetic information can be obtained from publicly available databases such as the GenBank at the National Center for Biotechnology Information (NCBI).

The Cas9 protein is an RNA-guided DNA endonuclease enzyme that induces double-stranded DNA breaks. For the Cas9 protein to accurately bind to the target nucleotide sequence and cleave the DNA strand, a short nucleotide sequence known as the PAM (Protospacer Adjacent Motif), consisting of three nucleotides, must be present next to the target sequence. The Cas9 protein cleaves between the third and fourth nucleotide pairs from the PAM sequence (NGG).

In the genome editing composition according to the present invention, the guide RNA and endonuclease protein can form a ribonucleic acid-protein (ribonucleoprotein) complex and function as an RNA-guided engineered nuclease (RGEN).

The present invention further provides a method for producing a genome-edited hot pepper plant with enhanced drought tolerance, comprising:
(a) introducing a guide RNA specific to a target nucleotide sequence of the *CaAIBZ1* (*Capsicum annuum* ASRF1-Interacting bZIP transcription factor 1) gene derived from hot pepper and an endonuclease protein into a hot pepper plant cell to edit the genome; and
(b) regenerating a hot pepper plant from the genome-edited hot pepper plant cell.

In the production method according to one embodiment of the present invention, the guide RNA specific to a target nucleotide sequence of the *CaAIBZ1* gene derived from hot pepper and endonuclease protein are as described in the above.

The CRISPR/Cas9 system used in the present invention is a gene editing method based on the NHEJ (non-homologous end joining) mechanism that introduces double-strand breaks at specific locations of the target gene for editing and induces insertion-deletion (InDel) mutations caused by imperfect repair during the DNA repair process.

In the production method according to the present invention, introducing a guide RNA and an endonuclease protein into a hot pepper plant cell in the step (a) may be using a complex (i.e., ribonucleoprotein) of a guide RNA specific to a target nucleotide sequence of the *CaAIBZ1* gene derived from hot pepper and an endonuclease protein; or a recombinant vector containing a DNA encoding a guide RNA specific to a target nucleotide sequence of the *CaAIBZ1* gene and a nucleic acid sequence encoding an endonuclease protein, but it is not limited thereto.

In the production method according to the present invention, the method of introducing the complex of guide RNA and endonuclease protein into plant cells (i.e., plant cell transformation) can be suitably selected from methods such as the calcium/polyethylene glycol method for protoplasts, electroporation of protoplasts, microinjection into plant cells, plant cell particle bombardment with DNA or RNA-coated particles, or *Agrobacterium tumefaciens*-mediated gene transfer, including infection by bacteria (incomplete transformation).

Additionally, introducing the recombinant vector containing a DNA encoding a guide RNA specific to a target nucleotide sequence and a nucleic acid sequence encoding an endonuclease protein into plant cells refers to a transformation method. Plant species transformation is now common not only for dicot plants but also for monocot plants. In principle, any transformation method can be used to introduce the recombinant vector according to the present invention into appropriate precursor cells.

In the production method according to the present invention, the "plant cells" into which the guide RNA specific to the target nucleotide sequence and the endonuclease protein are introduced may be any plant cells. Plant cells include cultured cells, cultured tissues, cultured organs, or entire plants. "Plant tissue" refers to differentiated or undifferentiated plant tissue, which includes, but is not limited to, roots, stems, leaves, pollen, spores, egg cells, seeds, and various forms of cells used for culturing, such as single cells, protoplasts, shoots, and callus tissues. Plant tissue can be *in planta* (in the plant) or in *ex vitro* conditions such as organ culture, tissue culture, or cell culture.

In the production method according to the present invention, the genome-edited hot pepper plant cell of the step (b) may be a plant cell having bi-allelic edited *CaAIBZ1* gene derived from hot pepper, but it is not limited thereto

In the production method according to the present invention, the method for regenerating a genome-edited plant from the genome-edited plant cell may employ any method known in the pertinent art. Techniques for regenerating mature plants from callus or protoplast culture are well-known in the pertinent art for a wide variety of plant species.

The present invention still further provides a genome-edited hot pepper plant with enhanced drought tolerance which is produced by the above method, and a genome-edited seed thereof.

The genome-edited hot pepper plant with enhanced drought tolerance according to the present invention is edited using the CRISPR/Cas9 system to modify the *CaAIBZ1* gene, which is involved in drought tolerance. Through bi-allelic knock-out of the *CaAIBZ1* gene, the genome-edited hot pepper plant earns a trait exhibiting enhanced drought tolerance compared to both non-edited hot pepper plants and mono-allelic *CaAIBZ1* gene-edited plants.

The present invention will be described in greater detail with reference to the following examples. However, the following examples are provided merely for illustrative purposes, and the content of the present invention is not limited to the following examples.

### EXAMPLES

### Example 1. Selection of sgRNA of CaAIBZ1 gene

The gRNA sequence targeting the hot pepper *CaAIBZ1* gene is shown in Table 1. The sgRNA listed in Table 1 was selected using the *CaAIBZ1* gene sequence (GenBank no: LY715037.1) and the CRISPR RGEN Tools website (www.rgenome.net/Cas-designer), considering various conditions such as GC content, out-of-frame score, mismatch number in the genome sequence, etc.

**[Table 1]**

| sgRNA Target sequence for *CaAIBZ1* gene editing | | | |
|---|---|---|---|
| Name | SEQ ID NO: | Nucleotide sequence (5'-3') | Direction |
| *CaAIBZ1* sgRNAl | 1 | ATCATTGTTGCCGATGTACTCGG | - |
| *CaAIBZ1* sgRNA2 | 2 | CTCAGTTCGTTGCCTCAAGAAGG | + |
| *CaAIBZ1* sgRNA3 | 3 | CACTACATCAAATTGGCATGTGG | + |
| *CaAIBZ1* sgRNA4 | 4 | TGCCGATGTACTCGGGCAACTGG | - |

To verify the editing efficiency of the selected four sgRNAs, each sgRNA was synthesized and mixed with Cas9 protein to form an RNP (ribonucleoprotein) complex. The complex was then introduced into hot pepper protoplast cells (1 x 10⁵ cells) using the PEG-mediated transformation method. After 24 hours, genomic DNA was extracted from the protoplasts, and Target deep sequencing analysis was carried out to assess the InDel efficiency.

The hot pepper protoplasts were isolated from the cotyledons of germinated seedlings of the C15 hot pepper line (Nongwoo Bio), following the method described by Yu et al. (Nat. Protoc. (2007) 2; 1565-1572), with slight modifications. Additionally, the RNP complex was prepared by mixing 30 µg of sgRNA and 30 µg of Cas9 protein with 1 µl of NEB buffer (#3), and incubating the mixture at room temperature for 10 minutes. After that, 10 to 20 µl of the RNP complex (sgRNA+Cas9) was added to the protoplasts, and an equal volume of 40% PEG solution was mixed and incubated at room temperature for 10 minutes.

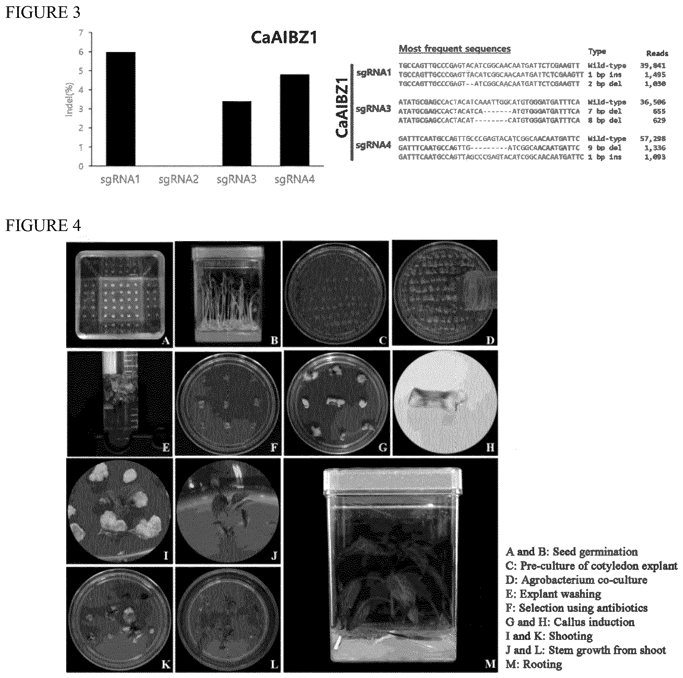

The analysis results showed that the target site editing efficiency was 6.0% for sgRNA 1, 0.0005% for sgRNA 2, 3.4% for sgRNA 3, and 4.8% for sgRNA 4 (Figure 3). Except for sgRNA 2, which showed no editing efficiency, the other sgRNAs were found to be suitable for targeting, and a recombinant vector for transformation was constructed using sgRNA 4.

### Example 2. Preparation of CaAIBZ1 gene-edited plant

The inventors of the present invention introduced the recombinant vector containing sgRNA4 into hot pepper cotyledon explants via *Agrobacterium-mediated* transformation. The process of regenerating plants from cotyledon explants was conducted by following, with some modifications, the method described by Lee et al. (Plant Cell Rep. 2004 Aug; 23(1-2): 50-8. doi: 10.1007/s00299-004-0791-1.).

### 2-1. Explant preparation and pre-culture

The hot pepper C15 line (Nongwoo Bio) was used in the experiment. The seeds were surface-sterilized by soaking in 95% ethanol for 30 seconds, followed by sterilization with 50% bleach solution for 10 minutes. After sterilization, the seeds were rinsed three times with sterile water. The sterilized seeds were then plated on 1/2 MS medium and germinated under light conditions at 25°C. After 8 to 10 days of germination, cotyledons and hypocotyls were excised from the plants and used as explants. The explants were wounded with a scalpel and then cultured on the pre-culture medium PM-A for 2 to 6 days under light conditions at 22°C to 28°C.

### 2-2. Co-culture with Agrobacterium

The recombinant vector was introduced into the *Agrobacterium* EHA105 strain. The transformed *Agrobacterium* was cultured in YEP medium containing 50 mg/L kanamycin, 50 mg/L rifampicin, and 100 µM acetosyringone. After centrifuging the culture, the culture broth was diluted with MS basic medium to an OD₆₀₀ value of 0.3 to 0.5. The culture suspension was then mixed with MS liquid medium (CCM medium from Table 2) containing 100 µM acetosyringone and 100 µM DTT (Dithiothreitol), and inoculated in the pre-cultured explants, which has been subjected to pre-culture in the above step 2-1, for 10 to 20 minutes. Afterward, the explants were co-cultivated in the same pre-culture medium PM-A used in the above step 2-1 under light conditions for 48 to 96 hours. The explants co-cultivated with *Agrobacterium* were washed three times with 1/2 MS liquid medium containing 3% sucrose, 600 mg/L timentin, and 2 mL/L PPM (Plant Preservation Mixture; Plant Cell Technology, USA).

### 2-3. Callus induction and shoot forming

To select a transformant, the explants co-cultivated with *Agrobacterium* in above Example 2-2 were cultured on MS basic medium (Selection-A medium from Table 2) containing 0.2 mg/L zeatin, 1.0 mg/L IAA, 100 mg/L kanamycin, 300 mg/L timentin, and 10 µM STS (silver thiosulfate + silver nitrate) for 4 to 6 weeks. After 4 to 5 weeks of culture, it was observed that some explants exhibit callus-like tissue formation around the wounded areas. To induce shoots from the callus, the explants were transferred to MS basic medium (TSIM medium from Table 2) containing 2 mg/L zeatin, 0.05 mg/L IAA, 50 mg/L kanamycin, 300 mg/L timentin, and 10 µM STS, and cultured for 8 to 12 weeks. Following that, to promote shoot growth, the explants were transferred again to MS basic medium (TEM medium from Table 2) containing 2 mg/L zeatin, 0.05 mg/L IAA, 2 mg/L GA3, 50 mg/L kanamycin, 300 mg/L timentin, and 10 µM STS, and cultured for 2 to 4 weeks.

### 2-4. Root induction and soil acclimation

The grown shoots were transferred to MS basic medium (TRIM medium from Table 2) containing 1 mg/L IBA (Indole-3-butyric acid) and 150 mg/L timentin for rooting induction and cultured for 2 to 4 weeks. After the regenerated plants developed roots, they were transferred to seedling pots containing horticultural soil and acclimated under 25°C, with a 16-hour light cycle, for 3 to 6 weeks.

**[Table 2]**

| **Composition of medium used for preparing hot pepper transformant** | | | |
|---|---|---|---|
| **Step** | **Media** | **Media Component** | **Culture Periods** |
| Germination | ½MS | ½MS Salt/Vitamin + sucrose 1.5% + agar 0.8%, pH 5.8 | 8-10 days under light |
| Pre-culture | PM-A | Basic media(MS + sucrose 3.0% + agar 0.8%, pH 5.8) + IAA 1.0 mg/L + zeatin 0.2 mg/L + 100 uM As(acetosyringone) | 2-6 days under light |
| Inoculation | CCM | MS salt + B5 Vitamin + sucrose 3.0% + 100 uM AS(acetosyringone) + 100 uM DTT, pH 5.8 * OD(600): 0.3 -0.5 | 2-4 days under light |
| Co-culture | Co | Pre-culture media A 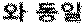 | |
| Selection (Callus induction) | Selection-A (PM-A + Km) | Agro Washing buffer (MS + sucrose 3.0%, pH 5.8 with timentin 600 mg/L + PPM 2 ml/L); | 4-6 weeks |
| | | Basic media + IAA 1.0 mg/L + zeatin 0.2 mg/L + kanamycin(Km) 100 mg/L + timentin(Tm) 300 mg/L + 10 uM STS(silver thiosulfate+Silver Nitrate) | |
| Shoot induction | TSIM | Basic media + zeatin 2.0 mg/L + IAA 0.05 mg/L + Km 50 mg/L + Tm 300 mg/L + 10 uM STS | 8-12 weeks |
| Shoot elongation | TEM | Basic media + (casein 200 mg/L) + zeatin 2.0 mg/L + IAA 0.05 mg/L + 2.0 mg/L GA₃ + Km 50 mg/L + Tm 300 mg/L + 10 uM STS | 2-4 weeks |
| Rooting | TRIM | ½ MS basic media + IBA 1.0 mg/L + Tm 150 mg/L | 3-6 weeks |

### Example 3. Determination of editing efficiency in hot pepper T₀ plant

To determine the editing efficiency in the obtained T₀ transformed plants, NGS analysis was carried out. As a result of the analysis of 35 regenerated CaAIBZ1-I4 hot pepper plants, 15 plants with over 10% editing efficiency were identified, including 3 mono-allelic level edited plants (50%) (i.e., number of plants showing an editing efficiency of at least 10% among the entire plants: 42.8%).

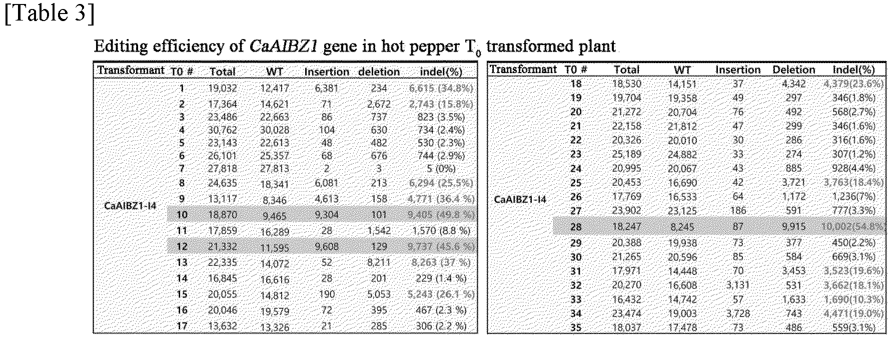

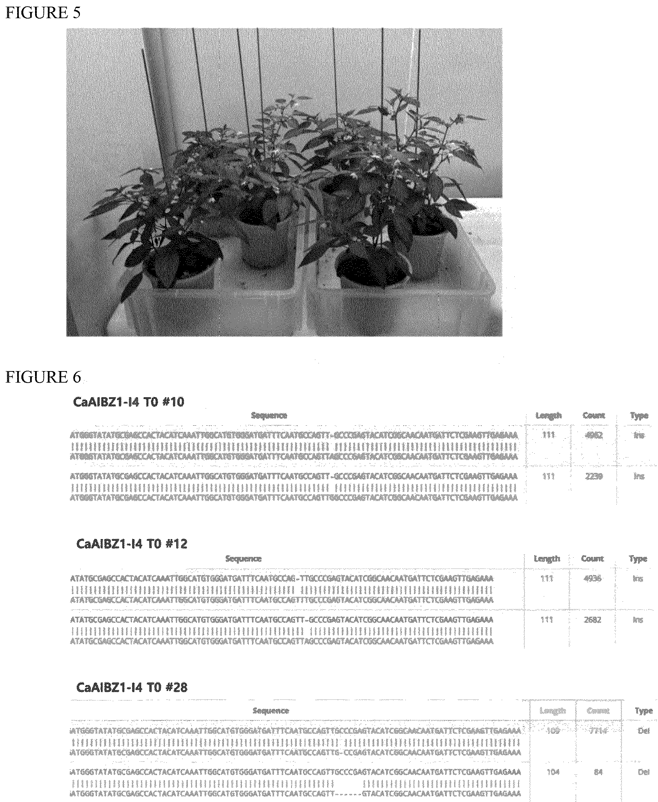

The CaAIBZ1-I4 T₀ #10 plant showed an overall editing efficiency of 49.8%, found as a mono-allelic edited plant (50%), with the main editing pattern being the insertion of a 1 bp A or G (Figure 6). The CaAIBZ1-I4 T₀ #12 plant showed an overall editing efficiency of 45.6%, found as a mono-allelic edited plant (50%), with the main editing pattern being the insertion of a 1 bp T or A (Figure 6). Both the CaAlBZ1-I4 T₀ #10 and CaAIBZ1-I4 T₀ #12 plants are expected to segregate in the next generation to yield edited plants with a 1 bp (single base) insertion. Additionally, the CaAIBZ1-I4 T₀ #28 plant showed an overall editing efficiency of 54.8%, found as a mono-allelic edited plant (50%), with the main editing pattern being predominantly a -1 bp deletion (Figure 6). The CaAIBZ1-I4 T₀ #28 plant is expected to segregate in the next generation to yield edited plants with a single base -1 bp deletion.

### Example 4. Determination of editing efficiency in hot pepper T₁ plant

NGS analysis was performed to determine the editing efficiency in the pot-planted T₁ hot pepper plant transformants. In the analysis of 42 regenerated CaAIBZ1-I4 hot pepper plants, 34 plants had editing efficiencies of 50% or higher including 12 plants found to have bi-allelic editing levels (93% or higher) (i.e., number of plants showing an editing efficiency of at least 50% among the entire plants: 81.0%).

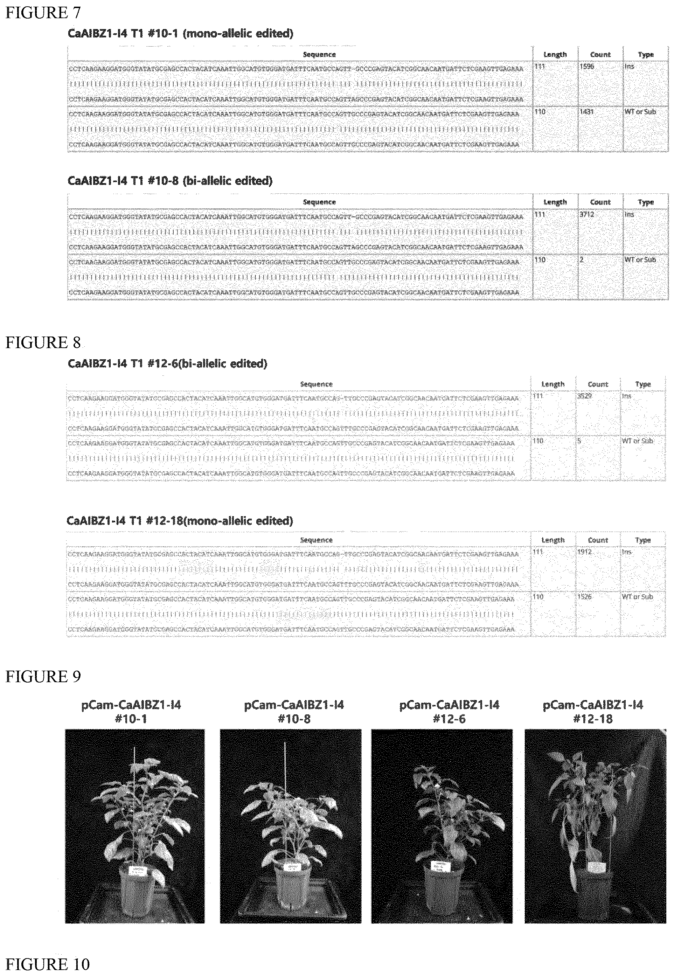

The editing efficiency of CaAIBZ1-I4 T₁ #10-8 and #12-6 plants was found at the bi-allelic editing level (99% or higher), while CaAIBZ1-I4 T₁ #10-1 and #12-18 plants showed mono-allelic editing levels (50%). The editing pattern in these plants showed that A or T base was inserted as +1 bp (Figures 7 and 8). This indicates that gene-edited plants with a +1 bp single base insertion were successfully obtained.

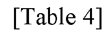

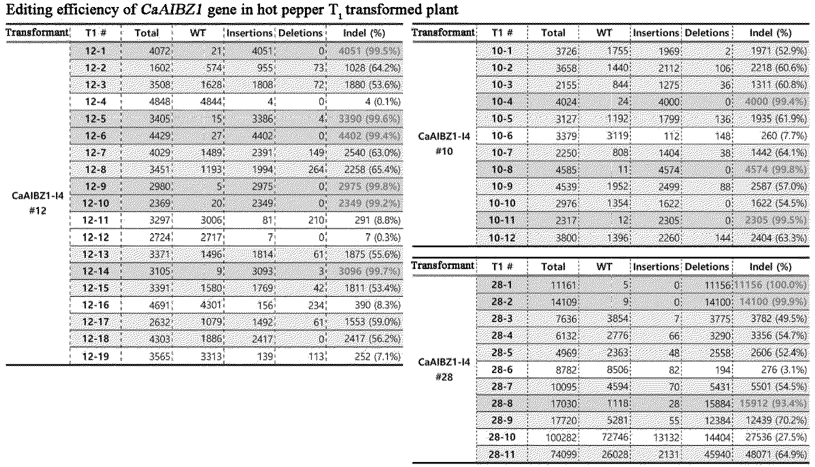

### Example 5. Analysis of drought tolerance of CaAIBZ1 gene-edited plants

To investigate the drought tolerance of the *CaAIBZ1* gene-edited hot pepper, seeds of four T₂ hot peppers with confirmed *CaAIBZ1* gene editing in the T₁ generation were sown along with the control (C15 line) (Figure 9). After 4 weeks of sowing, the gene-edited hot peppers were subjected to water withholding for 8 and 9 days, and the drought tolerance was observed. From the 6th day of the water withholding, the control group started to wilt, and by the 8th day, the plants were completely wilted (Figure 10). The mono-allelic gene-edited hot peppers showed similar drought tolerance to the control group, while the bi-allelic gene-edited hot peppers exhibited significantly stronger drought tolerance than the control group (Figure 10). On the 9th day of the water withholding, about 47% of the hot peppers completely survived (bi-allelic; 10-8 and 12-6), while the control group and mono-allelic hot peppers showed 0% survival (Figure 10).

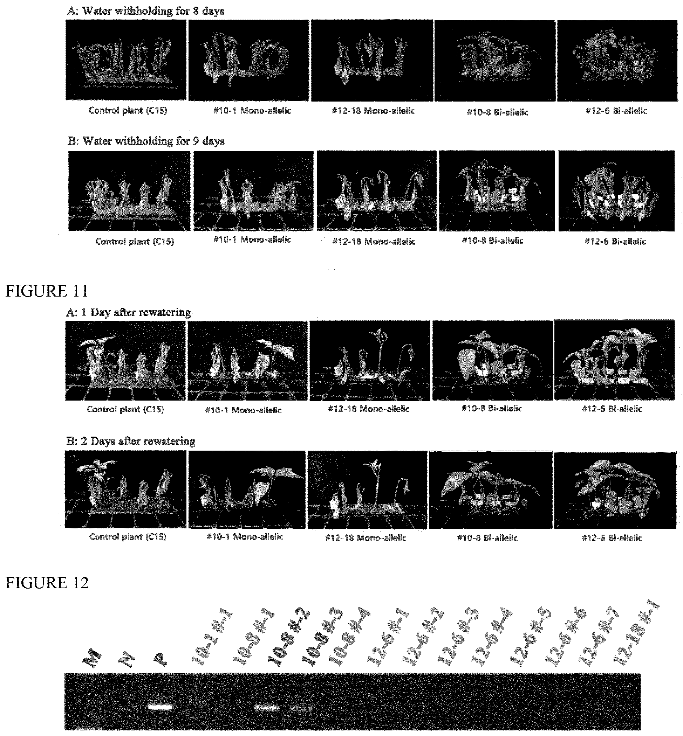

In addition, rewatering was started from the 10th day for both the control hot pepper plants and the gene-edited hot peppers that had been subjected to water withholding until the 9th day. In the control group, only one plant (20%) had recovered by the second day of rewatering. For the gene-edited hot peppers, the mono-allelic plants showed slower recovery compared to the bi-allelic plants. By the second day of rewatering, the full recovery rate was 25% for the mono-allelic edited plants and 73% for the bi-allelic edited plants (Figure 11).

Additionally, to determine the T-DNA free status in the 13 *CaAIBZ1* T₂ edited hot pepper plants selected as a plant showing the drought tolerance based on the above drought tolerance test (i.e., rewatering after water withholding), a PCR was performed using a Cas9 primer set [Cas9 #2-F: TTCGATAAGAACCTTCCAAA (SEQ ID NO: 5), Cas9 #2-R: TTGAGCCTTTCTTCAATCAT (SEQ ID NO: 6)]. As a result, PCR band was not detected in 11 out of the 13 plants, except for two plants (10-8 #2, 10-8 #3). Consequently, after advancing to the T₂ generation, 9 bi-allelic edited plants (10-8 #1, #4, and 12-6 #1-#7) having T-DNA removed from the *CaAIBZ1* gene-edited plants were obtained (Figure 12).

## Claims

1. A genome editing composition for enhancing drought tolerance in hot pepper plant, comprising as an active ingredient: a complex (i.e., ribonucleoprotein) of a guide RNA specific to a target nucleotide sequence of *CaAIBZ1* (*Capsicum annuum* ASRF1-Interacting bZIP transcription factor 1) gene derived from hot pepper and an endonuclease protein; or a recombinant vector containing a DNA encoding a guide RNA specific to a target nucleotide sequence of the *CaAIBZ1* gene and a nucleic acid sequence encoding an endonuclease protein.

2. The genome editing composition according to Claim 1, wherein the target nucleotide sequence of *CaAIBZ1* gene consists of the nucleotide sequence of SEQ ID NO: 1, SEQ ID NO: 3, or SEQ ID NO: 4.

3. A method for producing a genome-edited hot pepper plant with enhanced drought tolerance, comprising:
(a) introducing a guide RNA specific to a target nucleotide sequence of *CaAIBZ1* (*Capsicum annuum* ASRF1-Interacting bZIP transcription factor 1) gene derived from hot pepper and an endonuclease protein into a hot pepper plant cell to edit a genome; and
(b) regenerating a hot pepper plant from the genome-edited hot pepper plant cell.

4. The method according to Claim 3, wherein introducing a guide RNA and an endonuclease protein into a hot pepper plant cell in the step (a) uses a complex (i.e., ribonucleoprotein) of a guide RNA specific to a target nucleotide sequence of the *CaAIBZ1* gene derived from hot pepper and an endonuclease protein; or a recombinant vector containing a DNA encoding a guide RNA specific to a target nucleotide sequence of the *CaAIBZ1* gene and a nucleic acid sequence encoding an endonuclease protein.

5. The method according to Claim 3, wherein the target nucleotide sequence of *CaAIBZ1* gene consists of the nucleotide sequence of SEQ ID NO: 1, SEQ ID NO: 3, or SEQ ID NO: 4

6. A genome-edited hot pepper plant with enhanced drought tolerance which is produced by the method of any one of Claims 3 to 5.

7. The genome-edited hot pepper plant with enhanced drought tolerance according to Claim 6, wherein the genome-edited hot pepper plant is a bi-allelic *CaAIBZ1* gene-edited plant.

8. The genome-edited hot pepper plant with enhanced drought tolerance according to Claim 6, wherein the genome-edited hot pepper plant is T-DNA free.

9. A genome-edited seed of the hot pepper plant of Claim 6.
